# EUROPÄISCHE PATENTSCHRIFT

(11) **EP 0 837 050 B1**
(45) Veröffentlichungstag und Bekanntmachung des Hinweises auf die Patenterteilung: **23.08.2000**
(21) Anmeldenummer: 97117797.7
(22) Anmeldetag: 14.10.1997
(51) Int. Cl.: C07C 213/00, C07C 215/08

(54) **Verfahren zur heterogen katalysierten Herstellung von N-Hydroxyalkyl-substituierten Aminoalkinen**
Process for the preparation of N-hydroxyalkyl substituted aminoalkynes through heterogenous catalysis
Procédé de préparation d'aminoalkines N-hydroxyalkyl substitutées par catalyse hétérogène

(30) Priorität: 15.10.1996 DE 19642553
(43) Veröffentlichungstag der Anmeldung: 22.04.1998
(73) Patentinhaber: BASF AKTIENGESELLSCHAFT, 67056 Ludwigshafen (DE)
(72) Erfinder: Preiss, Thomas, Dr., 67065 Ludwigshafen (DE); Henkelmann, Jochem, Dr., 68165 Mannheim (DE); Wulff-Döring, Joachim, Dr., 67227 Frankenthal (DE); Stutz, Susanne, Dr., 69469 Weinheim (DE); Rühl, Thomas, Dr., 67227 Frankenthal (DE)
(74) Vertreter: Kinzebach, Werner, Dr.

(56) Entgegenhaltungen:
- CH-A- 414 594
- GB-A- 839 289
- US-A- 3 650 985

## Beschreibung

Die vorliegende Erfindung betrifft ein heterogen katalysiertes Verfahren zur Herstellung von N-Hydroxyalkyl-substituierten Aminoalkinen unter Verwendung eines heterogenen Kupferkatalysators.

N-Hydroxyalkyl-substituierte Aminoalkine sind wichtige industrielle Zwischenprodukte, die vielfach Verwendung finden. Sie dienen teilweise als Vorprodukte für Pharmazeutika, werden allerdings auch in der Galvanik oder als Korrosionsinhibitoren eingesetzt.

Die Herstellung von nicht Hydroxyalkyl-substituierten Aminoalkinen ist seit langem bekannt und wird industriell genutzt. Dabei werden im allgemeinen entsprechend substituierte Alkine, Carbonylverbindungen und Amine in einer homogen oder heterogen katalysierten Reaktion im Sinne einer Mannich-Kondensation umgesetzt.

Homogen katalysierte Verfahren dieses Typs sind weit verbreitet und vielfach beschrieben. So beschreibt z.B. die CH-A-669 192 die Herstellung von pharmakologisch wirksamen N-Arylalkyl-substituierten Aminoalkinen in einer homogen katalysierten Reaktion, wobei als Katalysatoren Kupfer- und Zinksalze, wie z.B. CuCl oder ZnCl₂ eingesetzt werden.

Die US-A-3,496,232 beschreibt die Herstellung von Propargylaminen aus Acetylen mit Hilfe der Mannich-Reaktion. Als Katalysatoren werden Salze von Metallen der ersten oder zweiten Nebengruppe, wie z.B. die Chloride, Acetate und Formiate von Kupfer beschrieben, die gegebenenfalls auch geträgert sein können. Bevorzugt erfolgt die Durchführung der Reaktion jedoch durch homogene Katalyse mit CuCl₂. Dieses Verfahren besitzt den Nachteil, daß es technisch aufwendig mit verflüssigtem Acetylen bei hohen Drücken (25 bis 70 atm) durchgeführt werden muß und keine zufriedenstellenden Ausbeuten an Produkt liefert.

Allgemein ergibt sich beim Arbeiten mit Acetylen und homogenen Kupferkatalysatoren das Problem, daß katalytisch aktive Kupferacetylide gebildet werden, die zur explosionsartigen Zersetzung neigen, nur schwer durch Filtration von der Reaktionslösung abzutrennen sind und zudem die Bildung von Cupren, einem Acetylenpolymerisationsprodukt, katalysieren.

Zur leichteren Handhabung von Kupferacetyliden werden diese auf einen inerten Träger aufgetragen und mit einer Bismutverbindung versetzt, um die Cuprenbildung zu verringern. Der Einsatz solcher bekannter Katalysatoren zur Aminoalkylierung von unter den Reaktionsbedingungen gasförmigen Alkinen erfordert jedoch hohe Partialdrücke um eine annähernd akzeptable Raum-Zeit-Ausbeute zu erzielen. Beim Arbeiten mit Acetylen, einem thermisch instabilen, bereits bei Normaldruck leicht explodierenden Gas, sind erhebliche sicherheitstechnische Maßnahmen zur Auslegung der Reaktoren für die erforderlichen Druckbereiche notwendig, was diese Verfahren wirtschaftlich benachteiligt.

So beschreibt z.B. die EP-A-0 080 794 ein heterogen katalysiertes Verfahren zur Herstellung von N,N-Di-(Alkyl, Phenyl)-substituierten Propinylaminen, wobei als bevorzugte Katalysatoren Kupferacetylide auf einem mit Bismutoxid dotierten Magnesiumsilikatträger eingesetzt werden. Die Reaktion erfolgt z.B. in einem Rührautoklaven mit einem aufgeschlämmten Katalysator oder in einem Festbett. Dieses Verfahren besitzt den Nachteil, daß der verwendete geträgerte Katalysator wegen seines geringen Kupfergehaltes (etwa 5 bis 35%) keine zufriedenstellende Aktivität besitzt. Die Umsetzung von Acetylen erfordert hierbei Partialdrücke von bis zu 20 atm oder mehr.

Die US-A-3,650,985 beschreibt die Herstellung trägerloser Kupferacetylid-Katalysatoren der allgemeinen Formel (CuC₂)_{w} (CH₂O)ₓ (C₂H₂)_{y} (H₂O)_{z} mit 1 ≤ w, x, y < 100, vorzugsweise w = 4, x = 0,24 bis 4, y = 0,24 bis 4 und z = 0,67 bis 2,8. Diese Katalysatoren können zusätzlich eine Bismutverbindung enthalten und sind durch simultanes Einwirken von Formaldehyd und Acetylen auf eine partikuläre, wasserunlösliche Kupferverbindung, vorzugsweise basisches Kupfercarbonat, wie z.B. synthetisch hergestellter Malachit herstellbar. Sie werden als wäßrige Katalysatoraufschlämmung zur Ethinylierung acetylenisch ungesättigter Kohlenwasserstoffe verwendet. Ähnliche Malachit-Katalysatoren werden in der US-A-3,560,576 beschrieben.

Die US-A-4,127,734 beschreibt die Herstellung von Bismut-modifizierten, kugelförmigen Malachiten und ihre Umsetzung mit Acetylen und Formaldehyd zu trägerlosen Ethinylierungskatalysatoren.

Weder in der US-A-3,650,985 noch in der US-A-4,127,734 wird jedoch vorgeschlagen, diese speziellen Katalysatoren in anderen Reaktionen zu verwenden. Insbesondere fehlt jeglicher Hinweis auf eine mögliche Brauchbarkeit dieser Katalysatoren für die Herstellung von Aminoalkinen in nichtwäßrigem Milieu.

Zudem findet der Fachmann in keiner der oben diskutierten Druckschriften einen Hinweis auf die Herstellung von Hydroxyalkyl-substituierten und speziell N-Hydroxyalkyl-substituierten Aminoalkinen.

Die DE-A-26 37 425 beschreibt die Herstellung von Dialkylamino-2-alkin-4-olen durch Umsetzung von Formaldehyd, Dialkylamin und einem Alkinol im Sinne einer Mannich-Kondensation. Um zufriedenstellende Ausbeuten zu erzielen, ist es jedoch erforderlich, spezielle Verfahrensbedingungen einzuhalten: Es muß in saurer Lösung, bevorzugt bei einem pH von 5, unter Verwendung eines speziellen Katalysatorsystems, nämlich einer Kombination von im Reaktionsgemisch löslichen Bromiden, Iodiden oder Jod und löslichen Cu(II)-Verbindungen gearbeitet werden. Die heterogen katalysierte Durchführung der Reaktion im neutralen oder alkalischen pH-Bereich wird nicht als mögliche Variante diskutiert.

Die DE-B-1 100 617 beschreibt ebenfalls die homogen katalysierte Herstellung von Dialkylamino-2-alkin-4-olen durch Umsetzung von Formaldehyd, Dialkylamin und einem Alkinol in wäßriger, saurer Lösung bei einem pH von 5 bis 6 und homogene Katalyse mit Kupfersulfat, -acetat, -nitrat oder -chlorid.

Auch in den beiden letztgenannten Druckschriften findet sich kein Hinweis auf die Herstellung von N-Hydroxyalkyl-substituierten Aminoalkinen.

Die GB-A-839 289 beschreibt acetylenisch ungesättigte Ethanolamine und ein Verfahren zu ihrer Herstellung. Dazu wird eine Acetylenverbindung, die mindestens ein aktives Wasserstoffatom besitzt, mit einem Oxazolidin der folgenden Formel: 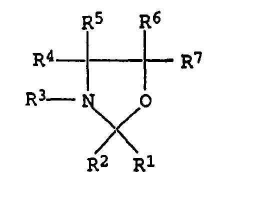 worin
- R¹, R², R⁴, R⁵, R⁶ und R⁷: gleich oder verschieden sind und für Wasserstoff, Alkyl, Aryl, Alkylaryl oder Arylalkyl stehen, unter der Maßgabe, daß entweder R¹ oder R² für Wasserstoff steht, und
- R³: für Alkyl, Hydroxyalkyl, Aryl, Alkylaryl oder Arylalkyl steht,
umgesetzt. Die Reaktion erfolgt lösungsmittelfrei oder in einem geeigneten Lösungsmittel, bevorzugt Dioxan oder Dimethylformamid, bei einer Temperatur zwischen 0 und 30°C, bei Über- oder Unterdruck, bevorzugt bei einem Druck oberhalb des Atmosphärendrucks und gegebenenfalls in Anwesenheit eines inerten Verdünnungsgases. Als Katalysatoren werden homogene Kupferkatalysatoren verwendet, z.B. Salze, wie Kupfersulfat oder Kupferchlorid, die im Reaktionsgemisch dissoziieren und mit dem Acetylen einen katalytisch wirksamen Komplex bilden. Die Abtrennung des Katalysators vom Reaktionsgemisch nach Beendigung der Reaktion erfolgt, indem man zuerst bereits ausgefallene Kupferacetylid-Komplexe abfiltriert, die dann noch in Lösung befindlichen Kupferionen mit Schwefelwasserstoff fällt und den entstandenen Niederschlag, gegebenenfalls unter Zugabe von Aktivkohle, abfiltriert. Diese Vorgehensweise ist nach den heute gültigen Sicherheitsstandards nicht mehr mit einem vertretbaren Aufwand durchzuführen. Wie zuvor erwähnt beschreibt die US-A-3,496,233 die Neigung dieser Kupferacetylid-Komplexe zur explosionsartigen Zersetzung und daß diese nur schwer durch Filtration von der Reaktionslösung abzutrennen sind. Die Fällung mit Schwefelwasserstoff, einem brennbaren, stark giftigen und in geringsten Konzentrationen übel riechenden Gas, das zudem starke Korrosionen an Metallen hervorruft, läßt sich gegenwärtig nicht mehr wirtschaftlich realisieren. Die dabei anfallenden schwerlöslichen Kupfersulfide müßten entweder aufwendig aufgearbeitet oder als Schwermetallabfall entsorgt werden. Die in der Schrift ebenfalls beschriebene Filtration unter Zuhilfenahme von Aktivkohle führt durch Adsorption von Produkt zu einer Ausbeuteverringerung.

Die CH-A-414 594 beschreibt die Herstellung von N-Hydroxyalkyl-substituierten Aminoalkinen durch Umsetzung eines Oxazolidins oder Tetrahydro-1,3-oxazins mit einer Acetylen-Verbindung in Gegenwart einer Kupferionenquelle und eines Lösungsmittels, bevorzugt Dioxan oder Dimethylformamid, bei einer Temperatur zwischen 0 und 30°C und einem Druck zwischen Atmosphärendruck und 40 cm Hg über Atmosphärendruck. Dabei wird ausschließlich Kupfer(I)chlorid als homogener Katalysator verwendet. Die Aufarbeitung erfolgt völlig analog zu dem in der GB-A-839 289 beschriebenen Verfahren und besitzt folglich auch dessen oben diskutierte Nachteile.

Shostakovskii et al. beschreiben in J. Org. Chem. USSR (Engl. Transl.), Bd. 6, Nr. 5, 1970, S. 902 ff die Herstellung von N-Hydroxyethyl-substituierten Aminopropinen durch Substitution eines Restes in N,N-disubstituierten Aminoethanolen mit 3-Brompropin in Gegenwart einer Base. Bei diesem Verfahren tritt als unerwünschte Nebenreaktion ein Ringschluß der Edukte unter Bildung von 2-Vinyloxazolidinen und weiteren ungesättigten Verbinmit einem 1,4-Oxazingerüst auf, so daß die Produktausbeuten zum Teil nur etwa 50% betragen.

Kukharev et al. beschreiben in J. APPL. CHEM. USSR (Engl. Transl.) EN, 63, 8.2, 1990, S. 1736 ff. die Synthese von N,N,N',N'-Tetrakis(2-hydroxyethyl)-1,4-diamino-2-butin durch Umsetzung von N-(2-Hydroxyethyl)oxazolidin mit Acetylen im Molverhältnis 2:1 bei einer Temperatur von 19 bis 24°C in Dioxan als Lösungsmittel. Als homogener Katalysator wird Kupferchlorid verwendet. Die Ausbeute beträgt dabei jedoch nur maximal 45%, wobei immer auch das Monokondensationsprodukt des Oxazolidins an Acetylen erhalten wird. Bei diesem Verfahren ist ebenfalls mit den vorbeschriebenen Problemen aufgrund der Verwendung von Kupferchlorid in einer homogen katalysierten Reaktion zu rechnen.

Der vorliegenden Erfindung liegt deshalb die Aufgabe zugrunde, ein Verfahren zur Herstellung von N-Hydroxyalkyl-substituierten Aminoalkinen zur Verfügung zu stellen, das die aus dem Stand der Technik bekannten Nachteile nicht mehr aufweist.

Überraschenderweise wurde nun gefunden, daß diese Aufgabe durch die Bereitstellung eines Verfahrens gelöst wird, bei dem ein Alkin mit einem 1-Oxa-3-azaheterocycloalkan in einer heterogen katalysierten Reaktion mit einem Katalysator, der eine Verbindung eines Metalls der ersten oder zweiten Nebengruppe umfaßt, umgesetzt wird. Überraschenderweise wurde weiterhin gefunden, daß das erfindungsgemäße Verfahren ein Arbeiten bei niedrigen Drücken ermöglicht, wenn ein unter den Reaktionsbedingungen gasförmiges Alkin, wie Acetylen, verwendet wird.

Gegenstand der vorliegenden Erfindung ist somit ein Verfahren zur Herstellung von N-Hydroxyalkyl-substituierten Aminoalkinen der allgemeinen Formel I 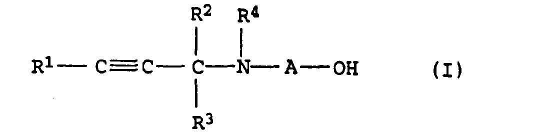 worin
- R¹: für ein Wasserstoffatom, Alkyl, Halogenalkyl, Cycloalkyl, Aryl, Alkoxy, Alkoxyalkyl, Hydroxyalkyl oder einen Rest der Formel

―CR²R³―NR⁴―A―OH

steht;
- R² und R³: unabhängig voneinander für ein Wasserstoffatom, Alkyl, Halogenalkyl, Cycloalkyl, Aryl, Alkoxy, Alkoxyalkyl oder Hydroxyalkyl stehen;
- R⁴: für ein Wasserstoffatom, Alkyl, Halogenalkyl, Cycloalkyl, Aryl, Alkoxy, Alkoxyalkyl oder Hydroxyalkyl steht;
- A: für einen C₂-C₅-Alkylenrest steht, der gegebenenfalls ein- oder mehrfach mit Alkyl, Halogenalkyl, Aryl, Alkoxy, Hydroxy und Hydroxyalkyl substituiert ist;
das dadurch gekennzeichnet ist, daß man ein Gemisch aus einem Alkin der allgemeinen Formel II

R¹―C≡C―H (II)

worin
- R¹: die oben angegebenen Bedeutungen besitzt,
und einem 1-Oxa-3-azaheterocycloalkan der allgemeinen Formel III 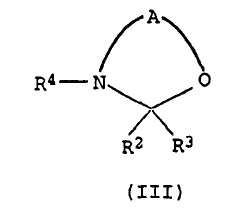 worin
- R², R³, R⁴ und A: die oben angegebenen Bedeutungen besitzen,
in einer heterogen katalysierten Reaktion unter Verwendung eines Katalysators, der eine Verbindung eines Metalls der ersten oder zweiten Nebengruppe umfaßt, umsetzt.

Im Rahmen der vorliegenden Erfindung steht Halogen für Fluor, Chlor, Brom und Jod und insbesondere für Chlor und Brom.

Der Ausdruck "Alkyl" umfasst geradkettige und verzweigte Alkylgruppen. Vorzugsweise handelt es sich dabei um geradkettige oder verzweigte C₁-C₁₂-Alkyl- und insbesondere C₁-C₆-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl und Dodecyl.

Halogenalkyl steht für eine wie oben definierte Alkylgruppe, die mit einem oder mehreren Halogenatomen, insbesondere Chlor und Brom, teilweise oder vollständig, vorzugsweise mit ein bis drei Halogenatomen, halogeniert ist.

Die obigen Ausführungen zur Alkylgruppe gelten in entsprechender Weise für die Alkylgruppe in Alkoxy-, Alkoxyalkyl- und Hydroxyalkyl-Resten.

Cycloalkyl steht vorzugsweise für C₃-C₈-Cycloalkyl, wie z.B. Cyclopropyl, Cyclobutyl, Cyclopentyl, Cyclohexyl, Cycloheptyl oder Cyclooctyl, oder Cyclopentylmethyl, Cyclopentylethyl und Cyclohexylmethyl und Cyclohexylethyl.

Aryl steht vorzugsweise für Phenyl oder Naphthyl.

Allgemein können für das erfindungsgemäße heterogen katalysierte Verfahren geträgerte und trägerlose Katalysatoren verwendet werden, wie sie auch für Ethinylierungsreaktionen verwendet werden.

Solche geträgerten Katalysatoren sind aus dem Stand der Technik bekannt und werden zum Beispiel in der US-A-4,119,790 und der EP-A-80 794 beschrieben, auf die hiermit in vollem Umfang Bezug genommen wird.

Bevorzugt wird im erfindungsgemäßen Verfahren eine Verbindung eines Metalls der ersten oder zweiten Nebengruppe des Periodensystems auf einem inerten Träger und insbesondere ein geträgerter Kupferacetylid-Katalysator verwendet, der aus einem Precursor hergestellt wird, welcher 10 bis 20 Gew.-% Kupferoxid, 1 bis 5 Gew.-% Bismutoxid sowie Siliciumdioxid als Trägermaterial umfaßt. Besonders bevorzugt verwendet man einen Kupferacetylid-Katalysator, der aus einem Precursor hergestellt wurde, der etwa 14 bis 15 Gew.-% Kupferoxid, etwa 4 Gew.-% Bi₂O₃ und etwa 80% SiO₂ umfaßt.

Geeignete trägerlose Katalysatoren umfassen einen Kupferacetylid-Komplex und zusätzlich eine Bismutverbindung, wie z.B. (BiO)₂CO₃, Bi(NO₃)₃ oder BiO(NO₃). Bevorzugt sind Katalysatoren mit etwa 40 bis 70 Gew.-% Cu und etwa 0,1 bis 10 Gew.-% Bi.

Solche Katalysatoren sind auch aus dem Stand der Technik bekannt und werden z.B. in der US-A-3,650,985, in der US-A-3,560,576 und in der US-A-4,127,734 beschrieben. Auf die Offenbarung dieser Druckschriften wird hiermit ausdrücklich Bezug genommen.

Gemäß einer bevorzugten Ausführungsform der vorliegenden Erfindung wird ein Katalysator verwendet, der mindestens einen Kupferacetylidkomplex der allgemeinen Formel IV

(CuC₂)_{w} (CH₂O)ₓ (C₂H₂)_{y} (H₂O)_{z} (IV)

mit 1 ≤ w, x, y, z < 100, und eine Bismutverbindung umfaßt.

Vorzugsweise besitzen dabei die Indices folgende Werte:
- w: 2 bis 6, insbesondere 4,
- x: 0,24 bis 4,00,
- y: 0,24 bis 2,40,
- z: 0,67 bis 2,8.

Verfahren zur Herstellung dieser gegebenenfalls Bi-dotierten Kupferacetylidkomplexe sind ebenfalls in der US-A-3,650,985 und in der US-A-4,127,734 beschrieben. Allgemein werden die erfindungsgemäß als trägerlose heterogene Katalysatoren verwendeten Kupferacetylidkomplexe durch gleichzeitige Reaktion einer Kupferverbindung, ausgewählt unter Kupferoxiden, Kupfersilikaten, Kupferphosphaten, Kupferhydroxiden und basischen Kupfercarbonaten, wie z.B. natürlichen und bevorzugt synthetischen Malachiten, in Gegenwart einer Bismutverbindung, ausgewählt unter Bismutoxidcarbonat und Bismutnitrat, und gegebenenfalls in Gegenwart eines Alkalimetallcarbonats oder -hydrogencarbonats, mit Formaldehyd und Acetylen erhalten.

Die Durchführung des erfindungsgemäßen Verfahrens kann in einem Rühr-, Rohr- oder Schlaufenreaktor als kontinuierlicher oder diskontinuierlicher Prozess erfolgen. Die Reaktionstemperatur liegt allgemein bei 0 bis 200°c, bevorzugt bei 20 bis 150°C, insbesondere bei 40 bis 120°C.

Der pH-Wert der Reaktion wird durch die Reaktanten vorgegeben und liegt im neutralen oder alkalischen pH-Bereich.

Die Reaktion kann lösungsmittelfrei oder in Gegenwart eines gegenüber den Reaktanten inerten organischen Lösungsmittels durchgeführt werden. Geeignete Lösungsmittel sind z.B. gesättigte cyclische Ether, wie z.B. Tetrahydrofuran und Dioxan.

Gemäß einer bevorzugten Ausführungsform des erfindungsgemäßen Verfahrens werden Alkine der Formel II umgesetzt, worin R¹ für ein Wasserstoffatom, Alkyl, oder Hydroxyalkyl steht.

Besonders eignet sich das erfindungsgemäße Verfahren zur Umsetzung von bei der jeweiligen Reaktionstemperatur gasförmigen Alkinen der Formel II, wie z.B. Acetylen, Propin, 1-Butin, etc. Vorzugsweise wird Acetylen verwendet.

Ist die Verbindung der Formel II Acetylen, so wird nach einer ersten Verfahrensvariante das 1-Oxa-3-azaheterocycloalkan der allgemeinen Formel III gemeinsam mit dem Katalysator und gegebenenfalls einem Lösungsmittel in einem Autoklaven vorgelegt. Es wird Acetylen aufgepreßt, bis ein Anfangsdruck von etwa 2 bis 8 bar, wie z.B. etwa 5 bar erreicht ist, und der Autoklav anschließend auf die Reaktionstemperatur erwärmt. Dann wird erneut Acetylen aufgepreßt, bis ein konstanter Druck von etwa 15 bis 25 bar, z.B. etwa 20 bar erreicht ist.

Vorteilhafterweise gelingt die Umsetzung von unter den Reaktionsbedingungen gasförmigen Alkinen nach einer zweiten Verfahrensvariante auch bei einem geringeren Druck als bei den aus dem Stand der Technik bekannten Verfahren, nämlich bei einem Druck von bis zu 3 bar, bevorzugt bis zu 2 bar und insbesondere bevorzugt bei Umgebungsdruck.

Wird nach der zweiten Verfahrensvariante Acetylen als Alkin der Formel II verwendet, so wird dieses für die Umsetzung vorzugsweise weder komprimiert noch verflüssigt. Das 1-Oxa-3-azaheterocycloalkan der Formel III wird gemeinsam mit dem Katalysator und gegebenenfalls einem Lösungsmittel in einem mit einer Mischvorrichtung versehenen Reaktor vorgelegt. Geeignete Reaktoren sind dem Fachmann bekannt. Zu ihnen zählen die in Ullmanns Enzyklopädie der technischen Chemie, 3. Aufl., Bd. 1, S. 117 ff und S. 769 ff (1951) beschriebenen Reaktionsbehälter für Reaktionen unter Druck. Die Zugabe des Alkins erfolgt vorzugsweise unterhalb des Flüssigkeitsspiegels der vorgelegten Reaktionsmischung, z.B. mit einem Tauchrohr oder mit einer Rohrschlange, die öffnungsbohrungen in der oder gegen die Strömungsrichtung der Reaktionsmischung aufweist. Die Zugabegeschwindigkeit wird durch die vorgenannten einzuhaltenden Druckbereiche limitiert. Vorzugsweise wird für die zweite Verfahrensvariante einer der vorgenannten trägerlosen Heterogenkatalysatoren verwendet.

Gemäß einer bevorzugten Ausführungsform steht die Verbindung der Formel III für ein 1-Oxa-3-azaheterocycloalkan der Formeln IIIa oder IIIb 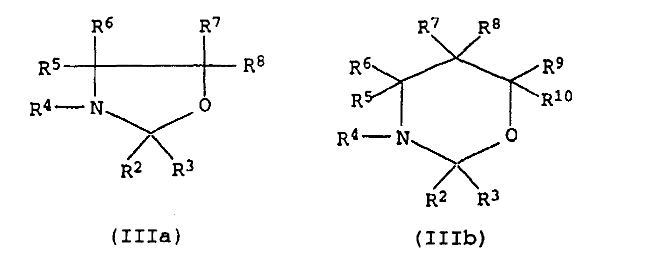 worin
R², R³ und R⁴ die zuvor angegebenen Bedeutungen besitzen,
R⁵, R⁶, R⁷, R⁸, R⁹ und R¹⁰ unabhängig voneinander für Wasserstoff, Alkyl, Halogenalkyl, Aryl, Alkoxy, Hydroxy oder Hydroxyalkyl stehen.

Besonders bevorzugt stehen R² und R³ unabhängig für ein Wasserstoffatom oder Alkyl. Insbesondere bevorzugt stehen R² und R³ beide für ein Wasserstoffatom.

R⁴ steht vorzugsweise für ein Wasserstoffatom, Alkyl oder Hydroxyalkyl.

Nach einer bevorzugten Ausführungsform stehen die Reste R⁵, R⁶, R⁷, R⁸, R⁹ und R¹⁰ unabhängig voneinander für Wasserstoff oder Alkyl. Bevorzugt stehen dabei einer, zwei oder drei der Reste R⁵, R⁶, R⁷, R⁸, R⁹ und R¹⁰ für Alkyl.

Verfahren zur Herstellung von Oxazolidinen der Formel IIIa und Tetrahydroxazinen der Formel IIIb sind dem Fachmann bekannt. So beschreibt die GB-A-839 289 die Herstellung von Oxazolidinen durch Kondensation von Ethanolamin mit einem Aldehyd oder Keton.

Die CH-A-414 594 beschreibt dazu analog die Synthese von Oxazolidinen und Tetrahydro-1,3-oxazinen aus gegebenenfalls substituierten Ethanol- und Propanolaminen und den entsprechenden Aldehyden. Auf diese Schriften wird hiermit in vollem Umfang Bezug genommen.

Die Erfindung wird anhand der folgenden, nicht einschränkenden Beispiele erläutert.

### Beispiele

### Beispiel 1

### Herstellung von N-Methyl-N-(2-hydroxypropyl-1)-3-aminopropin (R¹, R², R³ = H; R⁴ = CH₃; A = -CH₂-CH(CH₃)-)

In einem 100 ml Dreihalskolben wurden 10,1 g (0,1 mol) 3,5-Dimethyloxazolidin vorgelegt und mit 50 ml THF versetzt. Zu dieser Lösung wurden dann 1,5 g eines mit Acetylen aktivierten, trägerlosen Kupferkatalysators (54% Kupfer und 5% Bismut) gegeben und die Suspension 12 Stunden bei Raumtemperatur mit 6 1/Stunde Acetylen begast. Nach destillativer Aufarbeitung wurde das Monoadditionsprodukt in 85% Ausbeute (bezogen auf eingesetzten Heterocyclus) isoliert.

### Beispiel 2

### Herstellung von N,N-Bis-(2-hydroxyethyl-1)-3-aminopropin (R¹, R², R³ = H; R⁴ = C₂H₄OH; A = -(CH₂)₂-)

In einem 60 ml Autoklaven wurden 5,85 g (0,05 mol) N-Ethanol-1,3-oxazolidin vorgelegt und mit 30 ml THF versetzt. Zu dieser Lösung wurden dann 5 g eines mit Acetylen aktivierten geträgerten Kupferkatalysators (14 bis 15% CuO, 4% Bi₂O₃, 80% SiO₂) gegeben. Anschließend wurden 5 bar Acetylen aufgepreßt und die Lösung auf 50°C erwärmt. Es wurde dann weiter Acetylen bis zum Erreichen eines konstanten Druckes von 20 bar nachgepreßt. Nach destillativer Aufarbeitung konnte N,N-Diethanol-3-aminopropin in 80% Ausbeute (bezogen auf eingesetzten Heterocyclus) isoliert werden. Als Nebenprodukt wurden 18% N,N,N',N'-Tetraethanol-1,4-diaminobut-2-in isoliert.

### Beispiel 3

### Herstellung von N-Methyl-N-(3-hydroxypropyl-1)-3-aminopropin (R¹, R², R³ = H; R⁴ = CH₃; A = -(CH₂)₃-)

In einem 50 ml Zweihalskolben wurden 10,1 g (0,1 mol) N-Methyl-1,3-tetrahydrooxazin vorgelegt und mit 50 ml THF versetzt. Zu dieser Lösung wurden dann 1,5 g eines mit Acetylen aktivierten, trägerlosen Kupferkatalysators (54% Kupfer und 5% Bismut) gegeben und die Suspension 12 Stunden mit 6 1/Stunde Acetylen begast. Nach destillativer Aufarbeitung wurde das Monoadditionsprodukt in 78% Ausbeute (bezogen auf eingesetzten Heterocyclus) isoliert.

### Beispiel 4

### Herstellung von N,N-Bis-(2-hydroxyethyl-1)-3-aminopropin (R¹, R², R³ = H; R⁴ = C₂H₄OH; A = -(CH₂)₂-)

In einem 100 ml Dreihalskolben wurden 11,7 g (0,1 mol) N-Ethanol-1,3-oxazolidin vorgelegt und mit 50 ml THF versetzt. Zu dieser Lösung wurden dann 1,5 g eines mit Acetylen aktivierten, trägerlosen Kupferkatalysator (54% Kupfer und 5% Bismut) gegeben und die Suspension 12 Stunden mit 6 l/Stunde Acetylen begast. Nach destillativer Aufarbeitung wurde das Monoadditionsprodukt in 82% Ausbeute (bezogen auf eingesetzten Heterocyclus) isoliert.

## Patentansprüche

1. Verfahren zur Herstellung von N-Hydroxyalkyl-substituierten Aminoalkinen der allgemeinen Formel I 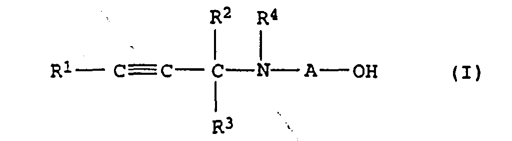 worin
R¹ für ein Wasserstoffatom, Alkyl, Halogenalkyl, Cycloalkyl, Aryl, Alkoxy, Alkoxyalkyl, Hydroxyalkyl oder einen Rest der Formel
―CR²R³―NR⁴―A―OH
steht;
R² und R³ unabhängig voneinander für ein Wasserstoffatom, Alkyl, Halogenalkyl, Cycloalkyl, Aryl, Alkoxy, Alkoxyalkyl oder Hydroxyalkyl stehen;
R⁴ für ein Wasserstoffatom, Alkyl, Halogenalkyl, Cycloalkyl, Aryl, Alkoxy, Alkoxyalkyl oder Hydroxyalkyl steht;
A für einen C₂-C₅-Alkylenrest steht, der gegebenenfalls ein- oder mehrfach mit Alkyl, Halogenalkyl, Aryl, Alkoxy, Hydroxy und Hydroxyalkyl substituiert ist;
dadurch gekennzeichnet, daß man ein Gemisch aus einem Alkin der allgemeinen Formel II
R¹―C≡C―H (II)
worin
R¹ die oben angegebenen Bedeutungen besitzt,
und einem 1-Oxa-3-azaheterocycloalkan der allgemeinen Formel III 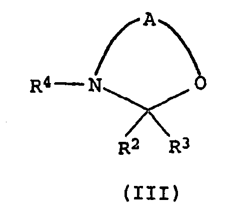 worin
R², R³, R⁴ und A die oben angegebenen Bedeutungen besitzen, in einer heterogen katalysierten Reaktion unter Verwendung eines Katalysators, der eine Verbindung eines Metalls der ersten oder zweiten Nebengruppe umfaßt, umsetzt.

2. Verfahren nach Anspruch 1, dadurch gekennzeichnet, daß der heterogene Katalysator ein Kupferkatalysator ist.

3. Verfahren nach Anspruch 2, dadurch gekennzeichnet, daß der heterogene Kupferkatalysator ein trägerloser oder geträgerter Katalysator ist.

4. Verfahren nach Anspruch 3, dadurch gekennzeichnet, daß ein Bismut-haltiger Katalysator verwendet wird, wobei die Katalysatormasse und/oder der Träger mit Bismut dotiert ist.

5. Verfahren nach Anspruch 4, dadurch gekennzeichnet, daß die Katalysatormasse 40 bis 70 Gew.-% Kupfer und 0,1 bis 10 Gew.-% Bismut enthält.

6. Verfahren nach einem der Ansprüche 1 bis 3, dadurch gekennzeichnet, daß der Kupferkatalysator von Malachit abgeleitet ist.

7. Verfahren nach einem der vorhergehenden Ansprüche, dadurch gekennzeichnet, daß als Kupferkatalysator ein Kupferacetylid-Katalysator verwendet wird.

8. Verfahren nach Anspruch 7, dadurch gekennzeichnet, daß der Katalysator mindestens einen Kupferacetylid-Komplex der allgemeinen Formel IV
(CuC₂)_{w} (CH₂O)ₓ (C₂H₂)_{y} (H₂O)_{z} (IV)
mit 1 ≤ w, x, y, z < 100,
enthält.

9. Verfahren nach Anspruch 8, dadurch gekennzeichnet, daß man einen Komplex der Formel IV verwendet, worin
w für einen Wert von 2 bis 6 steht,
x für einen Wert von 0,24 bis 4,00 steht,
y für einen Wert von 0,24 bis 2,40 steht, und
z für einen Wert von 0,67 bis 2,8 steht.

10. Verfahren nach einem der vorhergehenden Ansprüche, dadurch gekennzeichnet, daß man ein Alkin der Formel II umsetzt, worin R¹ für ein Wasserstoffatom, Alkyl oder Hydroxyalkyl steht.

11. Verfahren nach Anspruch 10, dadurch gekennzeichnet, daß als Verbindung der Formel II ein bei der Reaktionstemperatur gasförmiges Alkin verwendet wird.

12. Verfahren nach Anspruch 11, dadurch gekennzeichnet, daß als Verbindung der Formel II Acetylen verwendet wird und die Reaktion bei einem Acetylendruck von bis zu etwa 20 bar durchgeführt wird.

13. Verfahren nach einem der vorhergehenden Ansprüche, dadurch gekennzeichnet, daß man als Verbindung der Formel III ein 1-Oxa-3-azaheterocycloalkan der Formeln IIIa oder IIIb verwendet 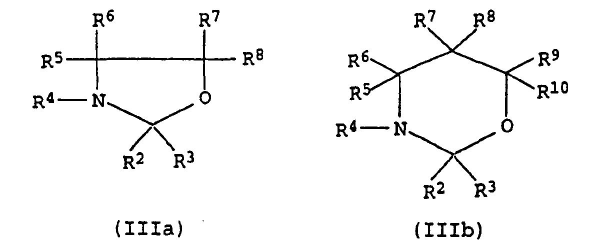 worin
R², R³ und R⁴ die zuvor angegebenen Bedeutungen besitzen,
R⁵, R⁶, R⁷, R⁸, R⁹ und R¹⁰ unabhängig voneinander für Wasserstoff, Alkyl, Halogenalkyl, Aryl, Alkoxy, Hydroxy oder Hydroxyalkyl stehen.

14. Verfahren nach einem der vorhergehenden Ansprüche, dadurch gekennzeichnet, daß die Umsetzung bei einer Temperatur von 0 bis 200°C, bevorzugt 20 bis 150°C, insbesondere 40 bis 120°C erfolgt.

15. Verfahren nach einem der vorhergehenden Ansprüche, dadurch gekennzeichnet, daß die Umsetzung im neutralen oder alkalischen pH-Bereich erfolgt.

16. Verfahren nach einem der vorhergehenden Ansprüche, dadurch gekennzeichnet, daß die Umsetzung im nichtwäßrigen Milieu oder in Substanz erfolgt.

## Claims

1. A process for preparing N-hydroxyalkyl-substituted aminoalkynes of the general formula I 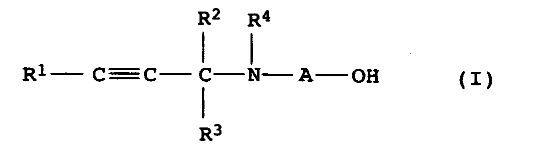 where
R¹ is hydrogen, alkyl, haloalkyl, cycloalkyl, aryl, alkoxy, alkoxyalkyl, hydroxyalkyl or
―CR²R³―NR⁴―A―OH;
R² and R³ are, independently of one another, hydrogen, alkyl, haloalkyl, cycloalkyl, aryl, alkoxy, alkoxyalkyl or hydroxyalkyl;
R⁴ is hydrogen, alkyl, haloalkyl, cycloalkyl, aryl, alkoxy, alkoxyalkyl or hydroxyalkyl;
A is C₂-C₅-alkylene which is unsubstituted or substituted one or more times by alkyl, haloalkyl, aryl, alkoxy, hydroxyl and hydroxyalkyl;
which comprises reacting a mixture of an alkyne of the general formula II
R¹―C≡C―H (II)
where
R¹ has the abovementioned meanings,
and a 1-oxa-3-azaheterocycloalkane of the general formula III 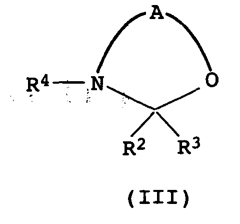 where
R², R³, R⁴ and A have the abovementioned meanings,
in a reaction with heterogeneous catalysis using a catalyst which comprises a compound of a metal of the first or second subgroup.

2. A process as claimed in claim 1, wherein the heterogeneous catalyst is a copper catalyst.

3. A process as claimed in claim 2, wherein the heterogeneous copper catalyst is an unsupported or supported catalyst.

4. A process as claimed in claim 3, wherein a bismuth-containing catalyst is used, the catalyst composition and/or the carrier being doped with bismuth.

5. A process as claimed in claim 4, wherein the catalyst composition comprises 40-70% by weight of copper and 0.1-10% by weight of bismuth.

6. A process as claimed in any of claims 1 to 3, wherein the copper catalyst is derived from malachite.

7. A process as claimed in any of the preceding claims, wherein a copper acetylide catalyst is used as copper catalyst.

8. A process as claimed in claim 7, wherein the catalyst comprises at least one copper acetylide complex of the general formula IV
(CuC₂)_{w} (CH₂O)ₓ (C₂H₂)_{y} (H₂O)_{z} (IV)
with 1 ≤ w, x, y, z < 100.

9. A process as claimed in claim 8, wherein a complex of the formula IV where
w is from 2 to 6,
x is from 0.24 to 4.00,
y is from 0.24 to 2.40, and
z is from 0.67 to 2.8,
is used.

10. A process as claimed in any of the preceding claims, wherein an alkyne of the formula II where R¹ is hydrogen, alkyl or hydroxyalkyl is reacted.

11. A process as claimed in claim 10, wherein an alkyne which is gaseous at the reaction temperature is used as compound of the formula II.

12. A process as claimed in claim 11, wherein acetylene is used as compound of the formula II, and the reaction is carried out under a pressure of up to about 20 bar of acetylene.

13. A process as claimed in any of the preceding claims, wherein the compound of the formula III used is a 1-oxa-3-azaheterocycloalkane of the formula IIIa or IIIb 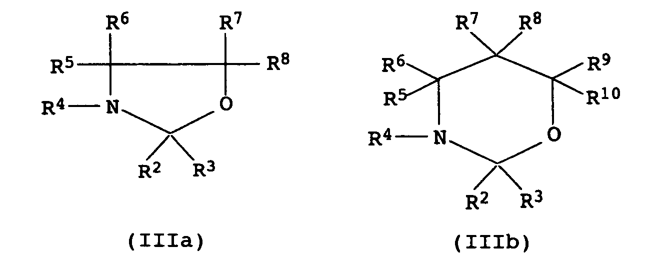 where
R², R³ and R⁴ have the abovementioned meanings,
R⁵, R⁶, R⁷, R⁸, R⁹ and R¹⁰ are, independently of one another, hydrogen, alkyl, haloalkyl, aryl, alkoxy, hydroxyl or hydroxyalkyl.

14. A process as claimed in any of the preceding claims, wherein the reaction is carried out at from 0 to 200°C, preferably 20 to 150°C, in particular 40 to 120°C.

15. A process as claimed in any of the preceding claims, wherein the reaction takes place in the neutral or alkaline pH range.

16. A process as claimed in any of the preceding claims, wherein the reaction takes place in a nonaqueous medium or without diluent.

## Revendications

1. Procédé de préparation d'amino-alcynes à substitution N-hydroxyalkyle de formule générale I 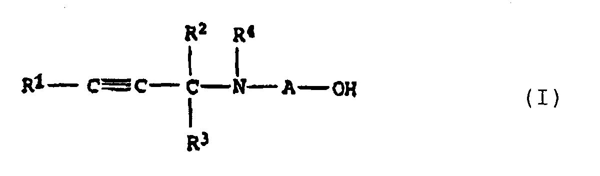 dans laquelle
R¹ représente un atome d'hydrogène, un groupe alkyle, halogénoalkyle, cycloalkyle, aryle, alcoxy, alcoxyalkyle, hydroxyalkyle ou un résidu de formule
-CR²R³-NR⁴-A-OH
R² et R³ représentent indépendamment l'un de l'autre un atome d'hydrogène, un groupe alkyle, halogénoalkyle, cycloalkyle, aryle, alcoxy, alcoxyalkyle ou hydroxyalkyle;
R⁴ représente un atome d'hydrogène, un groupe alkyle, halogénoalkyle, cycloalkyle, aryle, alcoxy, alcoxyalkyle ou hydroxyalkyle;
A représente un résidu alkylène en C₂ à C₅, qui est éventuellement mono- ou poly-substitué par un groupe alkyle, halogénoalkyle, aryle, alcoxy, hydroxy et hydroxyalkyle;
caractérisé en ce que l'on fait réagir un mélange d'un alcyne de formule générale II
R¹ - C ≡ C - H (II)
dans laquelle
R¹ prend les significations précitées,
et de 1-oxa-3-azahétérocycloalcane de formule générale III 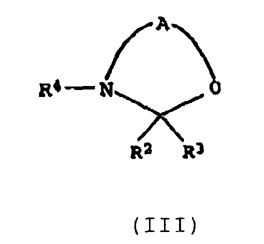 dans laquelle
R², R³, R⁴ et A prennent les significations précitées, dans une réaction à catalyse hétérogène, en utilisant un catalyseur qui comprend un composé d'un métal du premier ou deuxième sous-groupe du Tableau Périodique.

2. Procédé selon la revendication 1, caractérisé en ce que le catalyseur hétérogène est un catalyseur au cuivre.

3. Procédé selon la revendication 2, caractérisé en ce que le catalyseur hétérogène au cuivre est un catalyseur non supporté ou supporté.

4. Procédé selon la revendication 3, caractérisé en ce qu'un catalyseur contenant du bismuth est employé, dans lequel la masse du catalyseur et/ou le support sont dopés avec du bismuth.

5. Procédé selon la revendication 4, caractérisé en ce que la masse de catalyseur contient 40 à 70% en poids de cuivre et 0,1 à 10% en poids de bismuth.

6. Procédé selon l'une quelconque des revendications 1 à 3, caractérisé en ce que le catalyseur au cuivre est dérivé de la malachite.

7. Procédé selon l'une quelconque des revendications précédentes, caractérisé en ce qu'un catalyseur à acétylure de cuivre est employé comme catalyseur au cuivre.

8. Procédé selon la revendication 7, caractérisé en ce que le catalyseur contient au moins un complexe à acétylure de cuivre de formule générale IV
(CuC₂)_{w} (CH₂O)ₓ (C₂H₂)_{y} (H₂O)_{z} (IV)
avec 1 ≤ w, x, y, z < 100.

9. Procédé selon la revendication 8, caractérisé en ce que l'on emploie un complexe de formule IV, dans lequel
w vaut de 2 à 6,
x vaut de 0,24 à 4,00,
y vaut de 0,24 à 2,40, et
z vaut de 0,67 à 2,8.

10. Procédé selon l'une quelconque des revendications précédentes, caractérisé en ce que l'on fait réagir un alcyne de formule II, dans lequel R¹ représente un atome d'hydrogène, un groupe alkyle ou hydroxyalkyle.

11. Procédé selon la revendication 10, caractérisé en ce qu'un alcyne gazeux à la température de réaction est employé comme composé de formule II.

12. Procédé selon la revendication 11, caractérisé en ce que l'acétylène est employé comme composé de formule II et la réaction est réalisée à une pression d'acétylène allant jusqu'à environ 20 bar.

13. Procédé selon l'une quelconque des revendications précédentes, caractérisé en ce que l'on emploie, comme composé de formule III, un 1-oxa-3-azahétérocycloalcane de formule IIIa ou IIIb 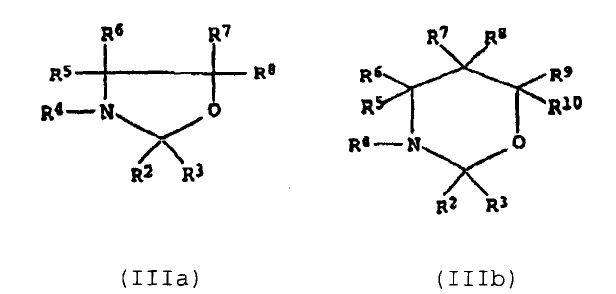 dans lesquelles
R², R³ et R⁴ prennent les significations précitées,
R⁵, R⁶, R⁷, R⁸, R⁹ et R¹⁰ représentent indépendamment l'un de l'autre, un atome d'hydrogène, un groupe alkyle, halogénoalkyle, aryle, alcoxy, hydroxy ou hydroxyalkyle.

14. Procédé selon l'une quelconque des revendications précédentes, caractérisé en ce que la réaction se déroule à une température de 0 à 200°C, de préférence de 20 à 150°C, en particulier de 40 à 120°C.

15. Procédé selon l'une quelconque des revendications précédentes, caractérisé en ce que la réaction se déroule dans la plage neutre ou basique des pH.

16. Procédé selon l'une quelconque des revendications précédentes, caractérisé en ce que la réaction se déroule dans un milieu non aqueux ou dans la masse.
